# EUROPÄISCHE PATENTSCHRIFT

(11) **EP 2 763 613 B1**
(45) Veröffentlichungstag und Bekanntmachung des Hinweises auf die Patenterteilung: **28.12.2016**
(21) Anmeldenummer: 12741029.8
(22) Anmeldetag: 02.08.2012
(51) Int. Cl.: A61B 17/70, A61B 17/86, A61B 17/00, A61B 90/00

(54) **NACHJUSTIERBARE POLYAXIALE PEDIKELSCHRAUBE**
READJUSTABLE POLYAXIAL PEDICLE SCREW
VIS PÉDICULAIRE POLYAXIALE AJUSTABLE

(30) Priorität: 05.10.2011 DE 102011054203
(43) Veröffentlichungstag der Anmeldung: 13.08.2014
(73) Patentinhaber: Aesculap AG, 78532 Tuttlingen (DE)
(72) Erfinder: PEUKERT, Andrea, 78532 Tuttlingen (DE)
(74) Vertreter: Winter, Brandl, Fürniss, Hübner, Röss, Kaiser, Polte - Partnerschaft mbB
(86) Internationale Anmeldenummer: PCT/EP2012/065196
(87) Internationale Veröffentlichungsnummer: WO 2013/050187

(56) Entgegenhaltungen:
- DE-A1- 19 507 141
- US-A1- 2005 187 548
- US-A1- 2005 267 472
- US-A1- 2010 114 180

## Beschreibung

Die vorliegende Erfindung betrifft allgemein eine polyaxiale Pedikelschraube und insbesondere eine nachjustierbare polyaxiale Pedikelschraube gemäß dem Oberbegriff des Patentanspruchs 1 sowie ein entsprechendes Wirbel-Stabilisierungssystem.

Pedikelschrauben dienen prinzipiell zur dorsalen Stabilisierung der Wirbelsäule bei Frakturen, Tumoren, Entzündungen, Deformitäten und degenerativ bedingten Instabilitäten mittels transpedikulärer Verschraubung. Dabei werden Pedikelschrauben in den Pedikeln jeweils benachbarter Wirbel platziert, worauf eine winkelstabile Verbindung zwischen den jeweils axial übereinander sich anordnenden Pedikelschrauben und einem axial sich erstreckenden Längsträger oder Steg geschaffen wird. Die Pedikelschrauben und Längsträger bilden dabei ein Wirbel-Stabilisierungssystem.

Hierfür hat eine Pedikelschraube in der Regel einen axialen, schaftartigen Außengewindeabschnitt, an den sich schraubenkopfseitig eine sogenannte Tulpe anschließt. Diese bildet konstruktiv eine U-förmig längs geschlitzte/getunnelte Aufnahmehülse mit Innengewinde, wobei die beiden sich gegenüberliegenden Längsschlitze jeweils einen Schlitzspalt vorbestimmter Spaltbreite definieren. In die parallel zueinander verlaufenden Längsschlitze ist der Längsträger eingelegt, der mittels einer Madenschraube oder Gewindemutter, die in das Innengewinde eingedreht ist, fixiert wird.

Grundsätzlich werden zwei Grundarten von Pedikelschrauben unterschieden, nämlich mono-axiale sowie polyaxiale Pedikelschrauben. Im Fall einer mono-axialen Pedikelschraube sind der Außengewindeabschnitt und die Tulpe einstückig miteinander ausgebildet, derart, dass sie fest miteinander verbunden, beispielsweise verschweißt oder verlötet sind. Eine polyaxiale Pedikelschraube hat hingegen einen als separates Schaftbauteil gefertigten Außengewindeabschnitt mit einem zumeist kugelförmigen oder (semi-) sphärischen Schraubenkopf, der von der hülsenförmigen Tulpe relativ verschwenkbar umgriffen und gleichzeitig im Übergangsbereich zwischen Kopf und Schaft hintergriffen wird. Auf diese Weise kann die Tulpe nach Versenken des Außengewindeabschnitts im Pedikelkanal eines Wirbels relativ zum Schaft verschwenkt und/oder verdreht werden, um eine gewünschte Lage und Ausrichtung im Wesentlichen unabhängig zur Ausrichtung des Schafts zu erhalten. Die Hinterschneidung verhindert dabei, dass die Tulpe vom Schaftkopf abgezogen werden kann. Anschließend wird die Tulpe mittels der Madenschraube bei zwischengelagertem Steg (Ein-Schrauben-Prinzip) oder durch eine zusätzliche Schraube/Schraubenmutter (Mehr-Schrauben-Prinzip) am Schraubenkopf lagefixiert.

Aus dem Stand der Technik, beispielsweise gemäß der EP 2 301 458 A1 ist eine polyaxiale Pedikelschraube gemäß dem Ein-Schrauben-Prinzip bekannt bestehend aus einem Schaftbauteil mit Außengewinde und sphärischem Kopf sowie einer U-förmig längs geschlitzten Aufnahmehülse (Tulpe) für einen Längsträger/Steg. Die Aufnahmehülse hat im Axialbereich hin zur Öffnung der Längsschlitze ein Innengewinde, in das eine Madenschraube eindrehbar ist und im Axialbereich hin zum jeweiligen Schlitzgrund einen radial nach innen gerichteten Umfangsvorsprung oder Absatz. Ferner ist in die Aufnahmehülse eine Art Kolben oder Stempel (auch als Inlay bezeichnet) mittels eines Sprengrings axialverschiebbar eingesetzt und dadurch jedoch gegen ein Herausfallen gesichert.

Zur Montage der aus der EP 2 301 458 A1 bekannten polyaxialen Pedikelschraube wird zunächst die Aufnahmehülse vom distalen (dem Schaftkopf gegenüberliegenden) Ende des Schaftbauteils her über dieses gestreift, solange, bis der radial nach innen ragende Absatz der Aufnahmehülse gegen den Schaftkopf (unterseitig) anschlägt. Anschließend wird der Stempel in die Aufnahmehülse (oberhalb des Schaftkopfs) eingedrückt, sodass der umfangsseitig zwischen Aufnahmehülse und Stempel sich anordnende Sprengring in entsprechende Umfangsnuten am Stempel und an der Aufnahmehülse einschnappt und die beiden Bauteile axial aneinander hält. Der Schaftkopf ordnet sich somit zwischen dem Absatz und dem Stempel (d.h. unterhalb des Stempels) an.

Sobald die Pedikelschraube in einen Wirbel eingedreht und fest darin verankert ist, wird ein Längsträger in den U-förmigen (Doppel-) Schlitz der Aufnahmehülse (oberhalb des Stempels) eingeführt, wobei sich die Aufnahmehülse relativ zum verankerten Schaftbauteil drehen und verschwenken kann. Auf diese Weise ist es einem Operateur möglich, die Aufnahmehülse entsprechend der Ausrichtung des Längsträgers anzupassen. Sobald die geeignete Relativlage der Aufnahmehülse eingestellt ist, wird die Madenschraube in die Aufnahmehülse soweit einschraubt, bis diese den Längsträger gegen den Stempel anlegt und diesen weiter in Axialrichtung der Aufnahmehülse gegen den Schaftkopf drückt. Auf diese Weise kann durch Festziehen der einzigen Madenschraube das gesamte Pedikelschrauben-Längsträger-System (Wirbel-Stabilisierungssystem) in der eingestellten Positionierung fixiert werden.

Wie in DE 195 07 141 A1 offenbart, kann eine Pedikelschraube gemäß dem Ein-Schrauben-Prinzip so ausgeformt sein, dass beim Fixiervorgang des Längsträgers die Tulpe gegenüber dem Schaftkopf mittels eines Arretierwerkzeugs temporär solange arretiert werden kann, bis diese Arretierung durch die Fixierschraube sichergestellt ist.

Die US 2011/0046683 A1 offenbart beispielsweise eine polyaxiale Pedikelschraube gemäß dem Mehr-Schrauben-Prinzip. Auch diese Pedikelschraube hat einen schaftartigen Außengewindeabschnitt mit einem integralen Schaftkopf an einem proximalen Ende des Schafts. Der Schaftkopf ist von einer Aufnahmehülse frei dreh- und schwenkbar umgeben, in der ebenfalls ein Innengewinde ausgebildet ist und die zwei U-förmige, sich gegenüberliegende Längsschlitze für einen Längsträger aufweist.

In die Aufnahmehülse ist ein Kolben/Stempel (Inlay) axial verschiebbar eingesetzt, der ebenfalls einen U-förmigen Längsschlitz in etwa mit den Schlitzbreiten-Abmessungen der Längsschlitze in der Aufnahmehülse aufweist.

Um die aus der US 2011/0046683 bekannte Pedikelschraube zu montieren, wird in bekannter Weise die Aufnahmehülse/Tulpe über den Schaft gestreift, bis diese an einem radial einwärts vorragenden Hülsenabsatz axial am Schaftkopf (unterseitig) schwenk- und drehbar anliegt. Hierauf wird der Stempel (oberhalb des Schaftkopfs) in die Aufnahmehülse eingesetzt und dessen U-förmiger Schlitz entsprechend den U-förmigen Schlitzen in der Aufnahmehülse ausgerichtet. Eine erste Schraube/Schraubenhülse wird daraufhin in die Aufnahmehülse eingeschraubt, die auf den Stempel (Inlay) unmittelbar einwirkt, um diesen ggf. gegen den Schaftkopf zu pressen. Diese erste Schraube/Schraubenhülse hat ein Innengewinde, in das eine zweite Schraube/Madenschraube eingedreht ist, die gegen einen quer in den Längsschlitz der Aufnahmehülse und des Stempels eingelegten Steg/Längsträger drückt, um diesen gegen den Stempel zu klemmen.

Soll nunmehr die Pedikelschraube gemäß der US 2011/0046683 in einem Wirbel gesetzt werden, wird hierfür der Schaft in den Wirbel eingedreht und anschließend die Aufnahmehülse relativ zum eingedrehten Schaft winkelausgerichtet. Zur Lagefixierung der Aufnahmehülse wird dann die erste Schraube angezogen, welche den Stempel (Inlay) unmittelbar gegen den Schaftkopf drückt und dadurch die Aufnahmehülse mit dem Schaftkopf verspannt. Abschließend wird ein Längsträger in den Längsschlitz zwischen Schaftkopf und erster Schraube quer eingelegt und mittels der zweiten Schraube gegen den Stempel geklemmt. Der Vorteil dieser Pedikelschraube gemäß der US 2011/0046683 besteht folglich darin, dass die Fixation der Polyaxialität und des Längsträgers unabhängig erfolgt, wofür jedoch eine vergleichsweise komplizierte Schraubenkonstruktion erforderlich ist.

Eine weitere polyaxiale Pedikelschrauben gemäß dem Mehr-Schrauben-Prinzip wird beispielsweise in US 2005/0267472 A1 offenbart. Bei einer Pedikelschraube gemäß US 2005/0267472 A1 kann die Tulpe gegenüber dem Schaftkopf mittels einer ersten Schraube fixiert werden. Mittels einer zweiten Schraube, die sich an der ersten Schraube abstützt, kann dann der Längsträger gegenüber dem Schaftkopf sowie der Tulpe fixiert werden.

In US 2005/0187548 A1 und US 2010/0114180 A1 werden Pedikelschrauben offenbart, bei denen die jeweilige Tulpe nicht mittels einer Schraube, sondern mithilfe andersartiger Verbindungsmittel gegenüber dem jeweiligen Schaftkopf fixiert werden kann. Bei der Pedikelschraube gemäß US 2005/0187548 A1 wird diese Fixierung mittels eines Fixierrings bewerkstelligt. Bei der Pedikelschraube gemäß US 2010/0114180 A1 sorgt eine Klemmkappe für die nötige Fixierung der Tulpe gegenüber dem Schaftkopf. Die jeweilige Fixierung des Längsträgers wird bei den Pedikelschrauben gemäß US 2005/0187548 A1 und US 2010/0114180 A1 wiederum durch Madenschrauben gewährleistet.

Bei sämtlichen genannten Pedikelschrauben sind darüber hinaus die Fixiermittel/Verriegelungselemente (Schrauben) im Wesentlichen selbsthemmend ausgeführt, um nach Implantierung kein ungewolltes Lösen der Längsträger von den Pedikelschrauben zu riskieren. Ferner sind die Fixationskräfte zwischen Pedikelschraube und Längsträger beträchtlich, da das gesamte System großen Belastungen standhalten muss, ohne dass sich die eingestellte Lagebeziehung zwischen Schaft, Hülse und Längsträger verändern darf. Diese Notwendigkeiten verursachen jedoch Probleme während des Implantierungsvorgangs.

Hat nämlich ein Operateur erst einmal das Verriegelungselement (beispielsweise die Madenschraube) mit Kraft festgezogen, ist die hierdurch festgelegte Lagebeziehung zwischen Aufnahmehülse und Schaft und/oder zwischen Aufnahmehülse und Längsträger nicht, oder nur mit großem Aufwand wieder veränderbar. In anderen Worten ausgedrückt, müsste hierfür der Operateur die mit großer Kraft festgezogene(n) Schraube(n) entgegen der Selbsthemmwirkung wieder lösen, ohne hierbei den im Wirbel bereits verankerten Außengewindeabschnitt mit zu lösen oder gar herauszubrechen. Des Weiteren wird durch das nachträgliche Lösen des Verriegelungselements (Schraube) dessen Selbsthemmwirkung ggf. beeinträchtigt, sodass die Funktionsfähigkeit der Pedikelschraube insgesamt nicht mehr gewährleistet ist. Die bekannten Systeme sind daher nicht oder nur bedingt Fehler verzeihend.

In Anbetracht der vorstehend geschilderten Problematik besteht eine Aufgabe der Erfindung darin, eine polyaxiale Pedikelschraube bereit zu stellen, deren Fixiermittel für ein Lösen und erneutes Herstellen eines Fixationszustands vorbereitet ist. Ein Ziel der Erfindung ist es hierbei, einem Operateur auch weiterhin zu ermöglichen, die Pedikelschraube in der gewohnten Art und Weise zu setzen, ohne gegenüber bekannten Schrauben zusätzliche OP-Schritte ausführen zu müssen. Ferner ist es ein Ziel der vorliegenden Erfindung, die Pedikelschraube und insbesondere deren Fixiermittel Fehler verzeihend auszubilden, derart, dass eine bereits fixierte Polyaxialität später wieder aufgehoben (entsperrt) und erneut fixiert (verriegelt) werden kann.

Die vorstehend genannte Aufgabe wird durch eine polyaxiale Pedikelschraube (vorzugsweise nach dem Ein-Schrauben-Prinzip) mit den Merkmalen des Anspruchs 1 sowie durch ein Wirbel-Stabilisierungssystem mit den Merkmalen des Anspruchs 16 gelöst. Vorteilhafte Ausgestaltungen der Erfindung sind Gegenstand insbesondere der Unteransprüche.

Der Kern der Erfindung besteht prinzipiell darin, das Fixiermittel der Pedikelschraube (bekannter Bauart wie vorstehend beschrieben) mit einem externen/separaten (zusätzlich betätigbaren) Gegenkraftbauteil auszurüsten, welches der Feststellkraft eines Verriegelungselements des Fixiermittels, beispielsweise der Madenschraube oder Gewindemutter entgegenwirkt und welches so gestaltet und/oder gehalten ist, dass die Feststellkraft des Verriegelungselements mittels des Gegenkraftbauteils wahlweise (durch manuelles Betätigen des Gegenkraftbauteils) freisetzbar (d.h. die Funktion des Verriegelungselements außer Kraft setzbar) ist.

Dieses Grundprinzip der Erfindung beruht demnach auf der Überlegung, das Verriegelungselement des Fixiermittels vorzugsweise bekannter Bauart für ein gewohntes Lagefixieren der eingestellten Polyaxialität zu verwenden, während sich das Gegenkraftbauteil des Fixiermittels in einem quasi eingekuppelten Zustand/Position befindet, in der das Verriegelungselement seine Funktion erfüllen kann. Um den Fixationszustand hingegen frei zu geben, soll nicht das Verriegelungselement (manuell) gelöst werden, sondern das Gegenkraftbauteil wird in einen quasi entkoppelten Zustand/Position gebracht bzw. geschaltet, in dem die Funktion des Verriegelungselements aufgehoben/reduziert wird. Dadurch ist es möglich, den Fixationszustand alleine durch Betätigen des Gegenkraftbauteils aufzuheben und wieder herzustellen, ohne dass das Verriegelungselement betätigt werden muss. Alternativ oder zusätzlich hierzu besteht aber auch die Möglichkeit, das Verriegelungselement im Wesentlichen kraftlos zu entriegeln, d.h. in dessen Entriegelungsposition zu bringen, ohne dass hierbei die Selbsthemmwirkung des Verriegelungselements beeinträchtigt wird (weil bereits im wesentlichen kraftlos). In diesem Fall kann dann das Gegenkraftelement zunächst kraftlos in seinen eingekuppelten Zustand erneut überführt werden, worauf dann das Verriegelungselement für ein Fixieren der Polyaxialität betätigt wird.

Etwas konkreter ausgedrückt wird das Verriegelungselement, beispielsweise eine Madenschraube, wie gewohnt direkt in die Tulpe eingedreht und drückt vorzugsweise über den eingesetzten Längsträger den Stempel axial gegen den Schaftkopf, um die Tulpe (und den Längsträger) vorzugsweise nach dem Ein-Schrauben-Prinzip mit dem Schraubenkopf zu verspannen. Das Gegenkraftelement bewirkt, dass die Verspannkraft auf den Schraubenkopf aufgebracht und/oder in die Tulpe abgeleitet wird. Wird das Gegenkraftelement außer Funktion gesetzt, weicht der Stempel und/oder das Verriegelungselement radial aus und die (axial wirkende) Verspannkraft wird abrupt aufgehoben.

Durch das vorstehend beschriebene Grundprinzip bleibt die Funktionsfähigkeit des Verriegelungselements prinzipiell erhalten. Gleichzeitig wird das Risiko eines Beschädigens oder Lösens der Verankerung der erfindungsgemäßen Pedikelschraube im Wirbel gegenüber dem Stand der Technik deutlich verringert.

Für das separate Gegenkraftbauteil kann es ferner grundsätzlich vorgesehen sein, dieses als Ein-Weg-Bauteil auszubilden, das folglich nur durch Zerstörung quasi entkoppelt und danach durch ein Austauschbauteil ersetzt wird. Alternativ kann das Gegenkraftbauteil mit einem Öffnungsmechanismus versehen sein, über den das Gegenkraftbauteil hinsichtlich seiner Abmessungen veränderbar ist, um so von einer Kupplungsform (-abmessung) in eine Entkupplungsform (-abmessung) reversibel überführbar zu sein. Auch ist es alternativ oder zusätzlich möglich, das Gegenkraftbauteil beweglich zu lagern, derart, dass es aus einer Funktionsposition (Gegenkraft erzeugende Position) in eine Außerfunktionsposition (keine Gegenkraft erzeugende Position) bewegbar ist.

Je nach Art der polyaxialen Pedikelschraube kann das Gegenkraftbauteil (ausschließlich nur) auf den Fixationsmechanismus des Fixiermittels zur Lagefixierung der Tulpe (Aufnahmehülse) bezüglich des Schafts und/oder auf den Fixationsmechanismus des Fixiermittels zur Lagefixierung der Tulpe bezüglich eines Längsträgers einwirken.

Ein Aspekt der Erfindung sieht vor, das Gegenkraftbauteil als ein Spann- oder Klemmmittel auszubilden, welches wahlweise in der Lage ist, die Form und/oder Funktion der Tulpe (Aufnahmehülse) und/oder des Stempels einer per se bekannten polyaxialen Pedikelschraube gemäß vorstehender Beschreibung aufrecht zu erhalten.

Vorzugsweise ist das Gegenkraftbauteil ein (Spann-) Ring, der vorzugsweise außenseitig um die Tulpe (Aufnahmehülse) und/oder den Stempel (im Bereich des Schaftkopfs) geführt ist und eine radial nach innen gerichtete (Gegen-) Kraft auf die Tulpe und/oder den Stempel aufbringen kann.

Weiter vorzugsweise ist der Ring axial verschiebbar gelagert, um in die Funktions- (im Axialbereich des Schaftkopfs) oder Außerfunktionsposition (versetzt zum Axialbereich des Schaftkopfs) wahlweise bringbar zu sein. Alternativ hierzu kann der Ring mit einem Öffnung- oder Weitenverstellmechanismus versehen sein, um den Ring quasi intern in die Funktions- oder Außerfunktionsposition wahlweise zu bringen. Die einfachste Alternative kann vorsehen, den Ring aus einem manuell einfach zerstörbaren Material zu fertigen oder mit einer manuell betätigbaren Sollbruchstelle auszubilden. So kann der Ring zerstört werden, ohne dass die Verankerung Schaden erleidet.

Vorteilhaft ist es, die Tulpe (Aufnahmehülse) und/oder den Stempel zumindest im Bereich des Gegenkraftbauteils, vorzugsweise in Form des Spann- und/oder Klemmmittels und weiter vorzugsweise des Spannrings mit federelastischen Eigenschaften auszubilden. Hierfür kann es vorgesehen sein, die Tulpe und/oder den Stempel mit Längsschlitzen auszuformen, wodurch elastisch radial nach außen aufbiegbare Laschen entstehen, welche vom Gegenkraftbauteil gemäß einer der vorstehenden Ausführungsformen radial wahlweise zusammengehalten werden. Alternativ ist es aber auch möglich, das Material der Tulpe und/oder des Stempels zumindest bereichsweise federelastisch zu machen. Diese Laschen haben vorzugsweise radial einwärts gerichtete Absätze (Klauen), welche den Schaftkopf hintergreifen und somit eine klemmende Axialkraft auf Schaftkopf übertragen können, dann, wenn der Spannring (in seiner Funktionsposition) ein radiales Aufspreizen des Laschen verhindert.

Im Fall der vorstehend genannten Axialverschiebung des Spannrings können zwei axial beabstandete Rastpositionen an der Tulpe und/oder dem Stempel ausgebildet sein, um die Funktions- oder Außerfunktionsposition zu markieren. Hierbei sei ausdrücklich darauf hingewiesen, dass die genannte Axialverschiebbarkeit des Spannrings auch durch eine Rotationsfähigkeit ersetzt werden kann um die Funktions- oder Außerfunktionsposition festzulegen.

Schließlich ist es grundsätzlich auch möglich, das Gegenkraftbauteil als eine einsetzbare und wahlweise auslösbare Gewindehülse auszubilden, die in der Tulpe und/oder dem Stempel verrastet ist. In diesem Fall überträgt die Gewindehülse die (Axial-) Kraft vom Verriegelungselement (Madenschraube) auf die Aufnahmehülse oder Stempel. Um das Verriegelungselement zu lösen, wird die Verrastung zwischen der Aufnahmehülse und der darin befindlichen Gewindehülse manuell gelöst und damit das Verriegelungselement kraftlos gemacht.

Die Erfindung wird nachstehend anhand eines bevorzugten Ausführungsbeispiels unter Bezugnahme auf die begleitenden Figuren näher erläutert.
Fig. 1a-1b zeigen jeweils die Perspektivenansicht eines Wirbel-Stabilisierungssystems gemäß einem bevorzugten Ausführungsbeispiel der Erfindung bestehend aus einer polyaxialen Pedikelschraube und einem schematisch dargestellten Längsträger in Funktionsposition,
Fig. 2 zeigt einen Längsschnitt des Wirbel-Stabilisierungssystems gemäß der Fig. 1,
Fig. 3a-3b zeigen jeweils die Perspektivenansicht des Wirbel-Stabilisierungssystems gemäß dem bevorzugten Ausführungsbeispiel der Erfindung in Außerfunktionsposition,
Fig. 4 zeigt einen Längsschnitt des Wirbel-Stabilisierungssystems gemäß der Fig. 3.
Fig. 5 zeigt die Aufnahmehülse in verriegeltem Zustand als vergrößerte Darstellung und
Fig. 6 zeigt die Aufnahmehülse in entriegeltem Zustand als vergrößerte Darstellung.

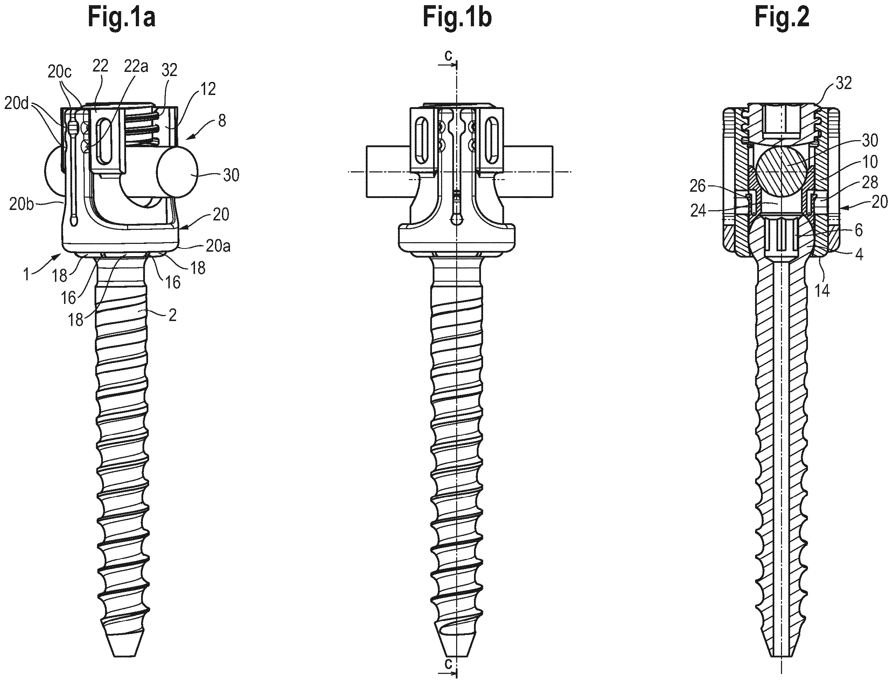

Das in den Fig. 1a-1b gezeigte Wirbel-Stabilisierungssystem gemäß dem bevorzugten Ausführungsbeispiel der Erfindung hat eine polyaxiale Pedikelschraube 1 bestehend aus einem mit einem Außengewinde versehenen Schaft oder Anker 2, an dessen einem proximalen Ende ein vorzugsweise sphärischer oder zumindest semi-sphärischer Schaftkopf 4 aus- oder angeformt ist. In den Schaftkopf 4 ist ein Werkzeugeingriff 6 beispielsweise für einen Innensechskant-Schraubenschlüssel, einen Kreuzschlitz-Schraubendreher, etc. eingearbeitet, um den Schaft 2 in den Pedikelkanal eines Wirbels (nicht dargestellt) einschrauben zu können.

Wie aus der Fig. 2 zu ersehen ist, bildet der Schaftkopf 4 an seinem Übergangsbereich zum geschraubten Schaft/Schaftabschnitt 2 eine in Axialrichtung wirkende Hinterschneidung. Diese entsteht vorliegend dadurch, indem der Schaftkopfdurchmesser größer gewählt ist als der Schaftdurchmesser. Alternativ wäre es aber auch denkbar, den Schaftkopf 4 durch eine Eindrehung (nicht gezeigt) am proximalen Endabschnitt des Schafts 2 vom übrigen geschraubten Schaftabschnitt 2 abzusetzen.

Gemäß der Fig. 1a-1b und 2 wird der Schaftkopf 4 von einer Aufnahmehülse 8 umgriffen, die (zunächst) dreh- und schwenkbar vom Schaftkopf 4 gehalten ist.

Im Konkreten hat die Aufnahmehülse 8 einen beidseits offenen zylindrischen Hülsenkörper 10, dessen Hülsenwand von einer proximalen Stirnseite her U-förmig aufgeschlitzt ist, wodurch sich eine im Wesentlichen lotrecht zur Hülsenachse verlaufende durchgehende Querrinne (Schlitz) 12 ergibt. Die Aufnahmehülse 8 ist im Axialbereich hin zur Offenseite des U-förmigen Schlitzes 12 mit einem Innengewinde ausgebildet, das sich gemäß der Fig. 2 in etwa bis zu einem axialen Mittenabschnitt der Hülse 8 erstreckt. Auf dem gegenüberliegenden Axialbereich (bzw. am axial distalen Ende) der Hülse 8, d.h. axial hinter dem Schlitzgrund des U-förmigen Schlitzes 12 ist die Aufnahmehülse 8 mit einem umlaufenden inneren Radialvorsprung/Absatz 14 ausgebildet, der den Hülseninnendurchmesser stirnseitig einengt und somit eine Hinterschneidung darstellt. Der Durchmesser des inneren Radialvorsprungs 14 ist dabei so bemessen, dass er geringfügig größer ist als der des geschraubten Schaftabschnitts 2 jedoch kleiner ist als der des Schaftkopfs 4. Ferner ist der Innendruchmesser der Hülse 8 geringfügig größer als der Durchmesser des Schaftkopfs 4.

Erfindungsgemäß ist die Aufnahmehülse 8 zumindest im Axialbereich ihres inneren Radialvorsprungs 14 mit zumindest in Radialrichtung wirkenden federelastischen oder flexiblen Eigenschaften ausgestattet. Insbesondere hat die Aufnahmehülse 8 an dem genannten distalen Endbereich eine Anzahl von in Umfangsrichtung beabstandeter Längsschlitze 16, wodurch sich eine Anzahl von laschen- oder zungenförmigen Hülsenabschnitten 18 ergeben, die sich in Axialrichtung parallel zueinander erstrecken und elastisch/flexibel radial nach Außen aufbiegbar sind. An dieser Stelle sei jedoch darauf hingewiesen, dass die genannten flexiblen Eigenschaften konstruktiv auch anders erzielbar sind, beispielsweise durch das axiale Ansetzen einer federelastischen Manschette (nicht gezeigt) an die Aufnahmehülse 8 oder durch Ausbilden einer offenen Gitter- oder Spiralstruktur (ebenfalls nicht gezeigt) in der Hülsenwand, etc.

Um die Aufnahmehülse 8 herum ist ein Gegenkraftbauteil 20 in der Form eines manschettenartigen Stellrings angeordnet, der axialverschieblich an/auf der Aufnahmehülse 8 geführt ist und den radial flexibel/elastisch aufspreizbaren distalen Endabschnitt der Aufnahmehülse 8 in einer ersten Position (Funktionsposition) anliegend umgreift.

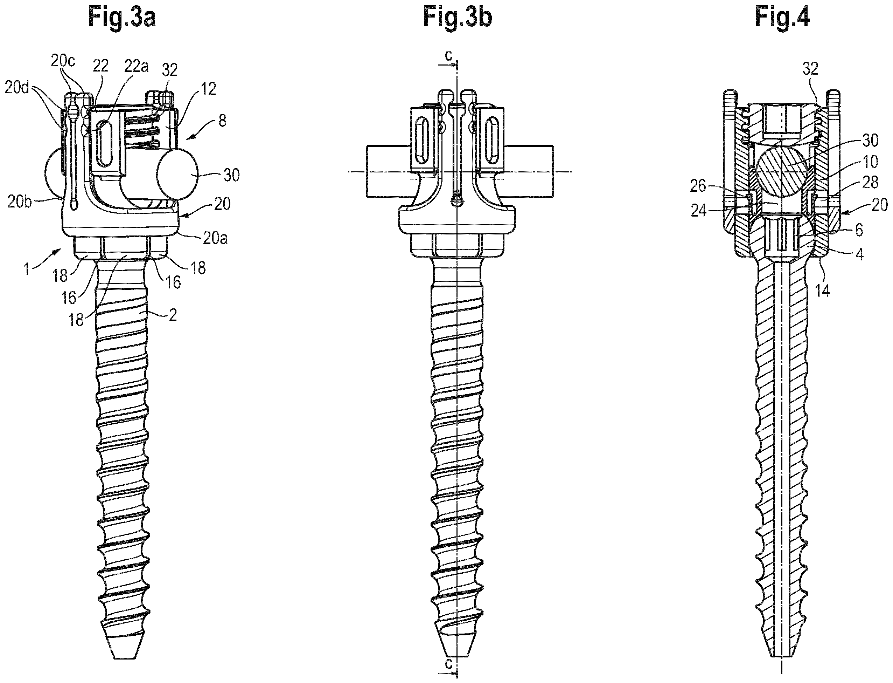

Im Einzelnen besteht der Stellring 20 aus einem in Umfangsrichtung geschlossenen Ringabschnitt 20a, an dessen einer zum Innengewindeabschnitt der Aufnahmehülse 8 hinweisenden Stirnseite zwei diametral sich gegenüberliegende Axialstege 20b vorzugsweise einstückig ausgeformt sind. Jeder Axialsteg 20b bildet dabei zwei axiale, parallele Federzungen 20c, die im vorliegenden Ausführungsbeispiel zwei axial beabstandete Hinterschneidungen oder Einkerbungen 20d aufweisen. Diese Einkerbungen 20d definieren hierbei im Betrieb eine Funktions- (gemäß Fig. 1a-1b und 2) und Außerfunktionsposition (gemäß Fig. 3a-3b und 4) des Stellrings 20. Im Gegenzug sind an der Außenseite der Aufnahmehülse 8 zwei diametral sich gegenüberliegende Axialnuten 22 an-/ausgeformt, in denen die zwei Axialstege 20b geführt sind und die zumindest einen Rastvorsprung (Noppe) 22a aufweisen, der mit den Einkerbungen 20d der Axialstege 20b in Wirkeingriff bringbar ist.

Alternativ zu der vorstehend beschriebenen sowie in den Fig. 1a-1b und 2 gezeigten Stellringkonstruktion ist es aber auch möglich, den Stellring 20 mit einer Sollbruchstelle (nicht dargestellt) auszubilden, an welcher der Stellring 20, der sich in diesem Fall grundsätzlich in der in Fig. 1a-1b gezeigten Funktionsposition befindet (d.h. unbeweglich ist), beispielsweise mittels eines Werkzeugs aufgebrochen werden kann. Auch ist es denkbar, die Axialverschiebbarkeit des Stellrings 20 mit einer Rotation zu kombinieren. In diesem Fall könnte anstelle der in der Fig. 1a-1b dargestellten Axialverrastung eine Art Bajonett-Verschluss (nicht gezeigt) vorgesehen sein, der durch Drehen des Stellrings 20 gelöst und daraufhin eine Axialverschiebung des Rings 20 längs der Aufnahmehülse 8 möglich ist.

Wie in der Fig. 2 dargestellt wird, ist in die Aufnahmehülse 8 ein Stempel oder Bolzen 24 eingesetzt, der sich im Axialbereich der flexiblen Laschen/Zungen 18 in der Hülse 8 anordnet. Der Stempel 24 ist hierbei mantelseitig mit zumindest einer federelastischen Rastnase 26 ausgebildet (gemäß der Fig. 2 sind zwei Rastnasen diametral angeordnet), die radial vorsteht und in Montageposition des Stempels 24 in eine radial ausgerichtete Ausnehmung 28 an der Innenseite der Aufnahmehülse 8 einrastet. Auf diese Weise bildet die zumindest eine Rastnase 26 eine Herausfallsicherung für den Stempel 24. Gleichzeitig ist die radiale Ausnehmung 28 so dimensioniert, dass sich der Stempel 24 um eine vorbestimmte Strecke axial innerhalb der Aufnahmehülse 8 bewegen kann.

An der dem Innenvorsprung 14 der Aufnahmehülse 8 zugewandten Stirnseite ist der Stempel 24 abgeflacht oder sphärisch, derart, dass er im Wesentlichen planar/flächig auf dem Schaftkopf 4 aufliegen kann. An der anderen Stirnseite ist der Stempel 24 rinnenartig ausgeformt, derart, dass ein rundstabförmiger Längsträger oder Steg 30 an dessen Mantelseite flächig mit dem Stempel 24 in Anlage bringbar ist. Schließlich zeigen die Fig.1a-1b und 2 noch ein Verriegelungselement in der Form einer Madenschraube (oder Setzschraube) 32, die in die Aufnahmehülse 8 an deren Innengewinde eingedreht ist, die auf den in den U-förmigen Schlitz vorliegend bereits eingeführten Längsträger 30 einwirken kann, um diesen gegen den Stempel 24 stirnseitig anzudrücken.

Die Funktionsweise der erfindungsgemäßen Pedikelschraube bzw. des Wirbelkörper-Stabilisierungssystems gemäß der Fig. 1a-1b bis 6 wird nachfolgend beschrieben.

Die Pedikelschraube 1a-1b gemäß der Erfindung wird mit dem Stellring 20 in der Funktionsposition gemäß der Fig. 1a-1b und 2 geliefert und eingesetzt. In dieser Funktionsposition umgreift der Stellring 20 die Federlaschen 18 der Aufnahmehülse 8 derart, dass sich diese radial nicht bzw. vernachlässigbar umbiegen/aufweiten lassen. Der Innendurchmesser der Aufnahmehülse 8 und damit des inneren Radialvorsprungs 14 ist damit festgelegt. In diesem Zustand umgreift die Aufnahmehülse 8 den Schaftkopf 4 drehsowie schwenkbar, wobei gleichzeitig der innere Radialvorsprung 14 den Schaftkopf 4 im Übergangsbereich zum geschraubten Schaftabschnitt 2 hinter-/untergreift. Somit kann von der Aufnahmehülse 8 eine Zugkraft auf den Schaftkopf 4 übertragen werden.

Soll die Pedikelschraube 1 gemäß der Erfindung implantiert werden, schraubt sie ein Operateur so in den Pedikelkanal eines Wirbels ein, wie er dies von herkömmlichen Pedikelschrauben gemäß eingangs beschriebenem Aufbau her gewöhnt ist. D.h. er verwendet hierfür ein (nicht gezeigtes) Schraubwerkzeug, das er in den Werkzeugeingriff 6 am Schaftkopf 4 einsetzt, um eine Schraubkraft unmittelbar auf den Schaft 2 aufzubringen. In diesem Montagestadium kann der Stempel 24, der mit einer axialen Durchgangsbohrung für ein Hindurchführen des Werkzeugs ausgebildet ist, bereits schon in die Aufnahmehülse 8 eingesetzt sein, sodass das Werkzeug durch den Stempel 24 hindurch auf den Werkzeugeingriff 6 des Schaftkopfs 4 eingreifen kann. Die Madenschraube 32 ist an dieser Stelle jedoch noch nicht eingedreht.

Sobald der geschraubte Schaft 2 fest in den Wirbel eingedreht (verankert) ist, wird der Stempel (Inlay) 24 in der Aufnahmehülse 8 eingerastet und die Madenschraube 32 über eine kurze Einschraubstrecke (vorzugsweise mittels eines Werkzeugs) so eingedreht, dass sich eine laterale Durchgangsöffnung zwischen dem Stempel 24 und der Madenschraube 32 in der Aufnahmehülse 8 ausbildet, deren Öffnungslänge demzufolge von der Position bzw. Einschraubstrecke der Madenschraube 32 abhängt.

Um nunmehr mehrere Pedikelschrauben der Erfindung, eingedreht an unterschiedlichen, axial beabstandeten Wirbeln, miteinander zu koppeln, wird der Längsträger 30 in der lateralen Durchgangsöffnung (d.h. den U-förmigen Schlitz 12) der Aufnahmehülse 8 (zwischen Stempel 24 und Schraube 32) eingeführt, wobei gleichzeitig die Aufnahmehülse 8 relativ zum Schaft 2 ausgerichtet wird (durch Drehen und Verschwenken). Alternativ zu der vorstehend beschriebenen Vorgehensweise ist es aber auch möglich und ggf. auch vorteilhaft, wenn zuerst der Längsträger 30 in die laterale Durchgangsöffnung der Aufnahmehülse 8 eingeführt wird, worauf dann die Madenschraube 32 (vorzugsweise mittels eines Werkzeugs) gesetzt wird. Bei dieser Montagevariante ergibt sich der Vorteil, dass die Durchgangsöffnung für das Einsetzen des Längsträgers 30 nicht unnötig durch das vorzeitige Setzen der Madenschraube 32 eingeengt wird.

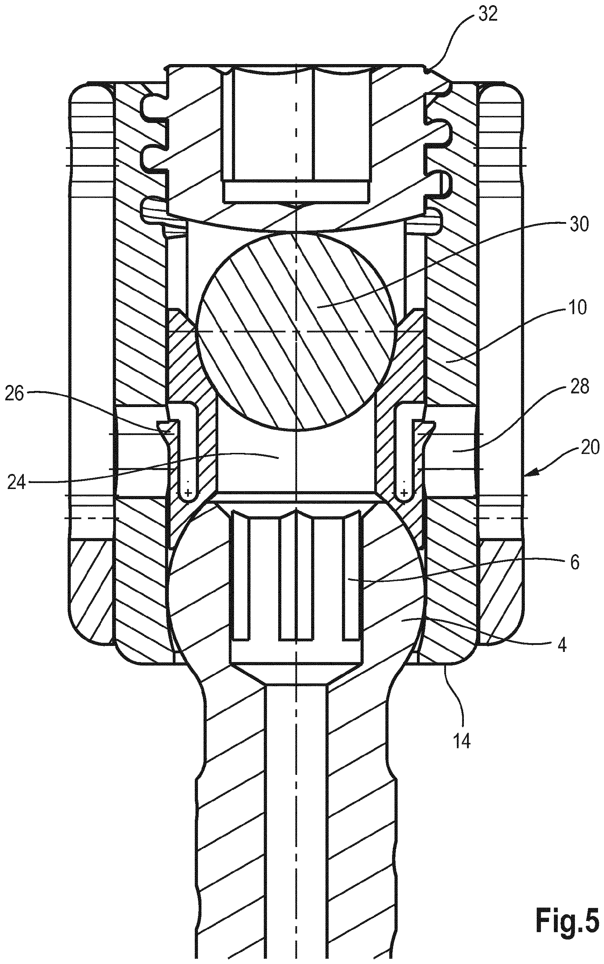

Nachdem der Längsträger 30 nach einer der vorstehenden Varianten eingeführt und die Madenschraube 32 gesetzt worden ist, wird die (Maden-) Schraube 32 mit dem (Schraub-)Werkzeug festgezogen. Dabei übt diese eine Druckkraft gegen den Längsträger 30 aus, die über den Stempel 24 weiter auf den Schaftkopf 4 als Widerlager übertragen wird. D.h. durch die Madenschraube 32 wird unter Einklemmen des Längsträgers 30 der Stempel 24 gegen den Schaftkopf 4 gedrückt, wodurch dieser zwischen dem inneren Radialvorsprung 14 der Aufnahmehülse 8 und dem Stempel 24 verspannt wird. Da sich hierbei die Hülse 8 infolge des im axialen Bereich des Radialvorsprungs befindlichen aussteifenden Stellrings 20 nicht aufspreizen kann (siehe insbesondere Fig. 5), lässt sich eine erhebliche Vorspannkraft auf den Schaftkopf 4 verwirklichen, die ausreicht, die Lageposition der Aufnahmehülse 8 zum Schaft 2 sowie die Position des Längsträgers 30 auch unter Belastung zu fixieren. Die Madenschraube 32 ist dabei so konzipiert, dass sich diese bei der zu erwartenden Belastung nicht mehr löst - also selbsthemmend wirkt. Darüber hinaus ist der Reibschluss zwischen der Aufnahmehülse 8 und dem Schaftkopf 4 dergestalt, dass selbst bei einem Lösen der Madenschraube 32 dieser Reibschluss nicht oder nur schwer frei kommt.

Damit wäre die Implantierung der erfindungsgemäßen Pedikelschraube 1 bzw. des Wirbel-Stabilisierungssystems abgeschlossen.

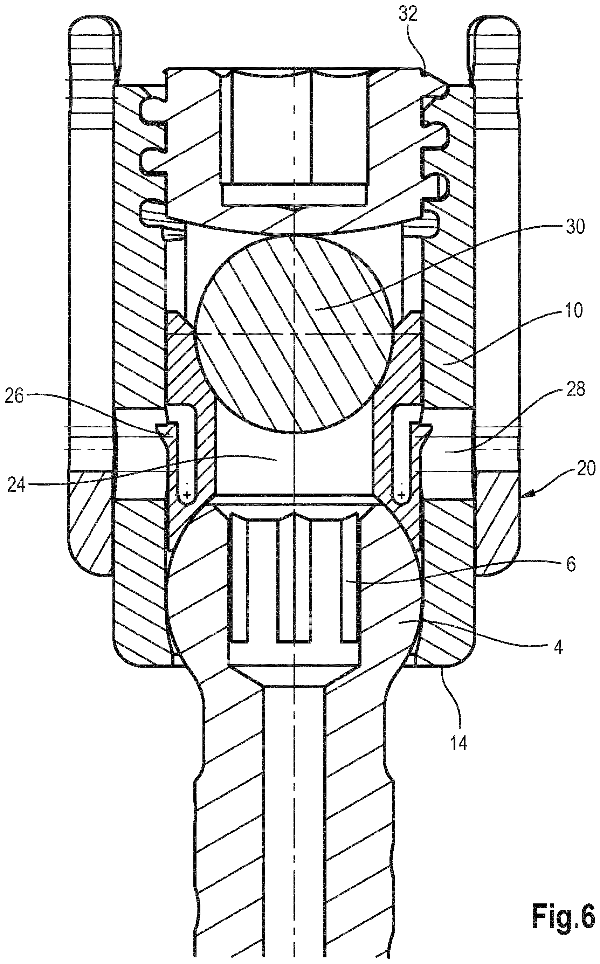

Jedoch kommt es vor, dass zu einem späteren Zeitpunkt der Sitz der Pedikelschraube 1 nochmals korrigiert werden muss. Zu diesem Zweck wird der Stellring 20 aus seiner in den Fig. 1a-1b und 2 gezeigten Funktionsposition (radial um den Radialvorsprung) in seine in den Fig. 3a-3b und 4 gezeigte Außerfunktionsposition (axial zum Radialvorsprung versetzt) geschaltet. Dieser Zustand wird insbesondere auch in Fig. 6 vergrößert gezeigt.

In anderen Worten ausgedrückt, wird der Stellring 20 manuell oder mit Hilfe eines nicht weiter gezeigten Hebelwerkzeugs längs der Außennuten 22 an der Aufnahmehülse 8 vorliegend in Richtung hin zur Madenschraube 32 verschoben, bis die durch die Kerben 20d in den Längsstegen 20b markierte Außerfunktionsposition erreicht ist. In dieser Außerfunktionsposition gemäß der Fig. 3a-3b und 4 umgreift der Stellring 20 nicht mehr oder nur noch unvollständig jenen Axialabschnitt der Aufnahmehülse 8, in denen die Längslaschen 18 daran ausgeformt sind, sodass sich diese flexibel, vorzugsweise elastisch radial aufbiegen können. Dadurch lässt sich der Innendurchmesser der Aufnahmehülse 8 und insbesondere der Innendurchmesser des inneren Radialvorsprungs 14 am distalen freien Ende der Laschen 18 in diesem Bereich aufweiten, wodurch der innere Radialvorsprung 14 der Aufnahmehülse 8 über den Schaftkopf 4 gleiten könnte. D.h. der Stellring 20, welcher als Gegenkraftbauteil zum Verriegelungselement (Madenschraube) 32 die Laschen 18 radial zusammengedrückt/-gehalten hat, verliert nunmehr diese Funktion, sodass die Einspannkraft der Madenschraube (Verriegelungselement) 32 freigegeben wird. Damit ist die Polyaxialität der Schraube 1 nicht mehr verriegelt.

In diesem Moment wirkt quasi keine Einspannkraft mehr auf die Madenschraube 32, sodass diese nahezu kraftlos aufgedreht werden kann. Soll die Pedikelschraube 1 und damit die Polyaxialität wieder verriegelt werden, muss lediglich der Stellring 20 aus seiner Außereingriffsposition im Wesentlichen kraftlos in die Funktionsposition gemäß vorstehender Definition verschoben werden, worauf die Madenschraube 32 erneut festgezogen werden kann. Dabei sei an dieser Stelle ausdrücklich darauf verwiesen, dass die Schraube 32 nicht notwendiger Weise betätigt werden muss. Vielmehr kann es bereits ausreichen, nur den Stellring 20 in seine Außereingriffsposition zu schalten, in der eine Relativbewegung der Tulpe zum Schaft möglich ist, um dann den Stellring 20 erneut in seine Eingriffsposition zu schalten (ohne Betätigung der Schraube), um die neue Relativlage zu fixieren.

Die vorliegende Erfindung betrifft abschließend zusammengefasst eine polyaxiale Pedikelschraube 1 mit dem geschraubten Schaftabschnitt 2 zur Verankerung der Pedikelschraube 1 in einem Wirbel, an dessen einem Axialende der Schaftkopf 4 ausgebildet ist, der mit der Aufnahmehülse 8 für einen Längsträger 30 dreh- und/oder schwenkbar gekoppelt ist, welche ein Fixiermittel (bestehend aus Verriegelungselement 32 und Gegenkraftbauteil 20) zur wahlweisen Lagefixierung der Aufnahmehülse 8 bezüglich des Schaftabschnitts 2 aufweist. Erfindungsgemäß hat das Fixiermittel ein Verriegelungselement, vorzugsweise eine (Maden-) Schraube 32 sowie ein separat zur Pedikelschraube 1 ausgebildetes (manuell betätigbares) Gegenkraftbauteil, vorzugsweise einen (verschiebbaren und/oder verdrehbaren) Stellring 20, welches der Feststellkraft des Verriegelungselements, vorzugsweise die Madenschraube 32 oder eine Gewindemutter, entgegenwirkt und welches so gestaltet und/oder gehalten ist, dass die Feststellkraft des Verriegelungselements 32 mittels des Gegenkraftbauteils 20 wahlweise freisetzbar, d.h. (nahezu) wirkungslos ist.

## Patentansprüche

1. Polyaxiale Pedikelschraube (1) mit einem geschraubten Schaftabschnitt (2) zur Verankerung der Pedikelschraube (1) in einem Wirbel, an dessen einem Axialende ein Schaftkopf (4) ausgebildet ist, der mit einer Aufnahmehülse (8) für einen Längsträger (30) dreh- und/oder schwenkbar gekoppelt ist, welche ein Fixiermittel (20, 32) zur wahlweisen Lagefixierung der Aufnahmehülse (8) bezüglich des Schaftabschnitts (2) aufweist, **dadurch gekennzeichnet, dass** das Fixiermittel (20, 32) ein Verriegelungselement (32) sowie ein separates Gegenkraftbauteil (20) hat, welches der Feststellkraft des Verriegelungselements (32), vorzugsweise einer Madenschraube oder Gewindemutter entgegenwirkt und welches so gestaltet und/oder gehalten ist, dass die Feststellkraft des Verriegelungselements (32) mittels des Gegenkraftbauteils (20) wahlweise freisetzbar ist.

2. Polyaxiale Pedikelschraube nach Anspruch 1, **dadurch gekennzeichnet, dass** das Verriegelungselement (32) für ein Lagefixieren der eingestellten Polyaxialität vorgesehen ist, wenn sich das Gegenkraftbauteil (20) in einem Kraft aufnehmenden Zustand oder Funktionsposition befindet, wohingegen das Gegenkraftbauteil (20) für ein Aufheben oder Reduzieren der Funktion des Verriegelungselements (32) vorgesehen ist, wenn sich dieses bereits in einem Fixationszustand befindet.

3. Polyaxiale Pedikelschraube nach Anspruch 1 oder 2, **dadurch gekennzeichnet, dass** das Gegenkraftbauteil (20) als manuell zerstörbares Ein-Weg Bauteil ausgebildet ist, und/oder mit einem Öffnungsmechanismus versehen ist, über den das Gegenkraftbauteil (20) hinsichtlich seiner Abmessungen veränderbar ist und/oder beweglich gelagert ist, derart, dass es aus einer Funktionsposition in eine Außerfunktionsposition bewegbar ist.

4. Polyaxiale Pedikelschraube nach einem der Ansprüche 1 bis 3, **dadurch gekennzeichnet, dass** das Gegenkraftbauteil (20) auf den Fixationsmechanismus des Fixiermittels zur Lagefixierung der Aufnahmehülse (8) bezüglich des Schaftabschnitts (2) und/oder auf den Fixationsmechanismus des Fixiermittels zur Lagefixierung der Aufnahmehülse (8) bezüglich eines Längsträgers (30) einwirkt.

5. Polyaxiale Pedikelschraube nach einem der Ansprüche 1 bis 4, **dadurch gekennzeichnet, dass** das Gegenkraftbauteil (20) als ein Spann- oder Klemmmittel ausgebildet ist, welches wahlweise in der Lage ist, die Form und/oder Funktion der Aufnahmehülse (8) und/oder eines Stempels (24) aufrecht zu erhalten, der zur Kraftübertragung der Verriegelungselements (32) auf den Schaftkopf (4) in der Aufnahmehülse (8) gelagert ist.

6. Polyaxiale Pedikelschraube nach einem der Ansprüche 1 bis 5, **dadurch gekennzeichnet, dass** das Gegenkraftbauteil (20) ein Spannring ist, der vorzugsweise außenseitig um die Aufnahmehülse (8) und/oder einen in der Aufnahmehülse axialverschieblich gelagerten Stempel (24) zur Kraftübertragung zwischen Verriegelungselement (32) und Schaftkopf (4) geführt ist und dafür vorgesehen ist, je nach eingestellter Form und/oder Position eine radial nach innen gerichtete Gegenkraft auf die Aufnahmehülse (8) und/oder den Stempel (24) aufzubringen, wobei der Spannring (20) insbesondere aus einem manuell mit vorbestimmtem Kraftaufwand unterhalb der Belastungsgrenze des Schaftabschnitts (2) zerstörbaren Material gefertigt oder mit einer manuell betätigbaren Sollbruchstelle ausgebildet ist.

7. Polyaxiale Pedikelschraube nach einem der Ansprüche 1 bis 5, **dadurch gekennzeichnet, dass** das Gegenkraftbauteil (20) eine Gewindehülse ist zur Aufnahme des Verriegelungselements (32), die in die Aufnahmehülse (8) wahlweise lösbar vorzugsweise durch einen Bajonett- oder Clipverschluss verrastet ist, um die Vorspannkraft des in der Gewindehülse angeordneten Verriegelungselements (32) auf die Aufnahmehülse (8) zu übertragen oder wahlweise freizugeben.

8. Polyaxiale Pedikelschraube nach Anspruch 6, **dadurch gekennzeichnet, dass** der Ring (20) axial verschiebbar an der Außenseite des Aufnahmehülse (8) gelagert ist, um in die Funktions- oder Außerfunktionsposition wahlweise bringbar zu sein.

9. Polyaxiale Pedikelschraube nach Anspruch 6, **dadurch gekennzeichnet, dass** der Ring (20) mit einem Öffnung- oder Weitenverstellmechanismus versehen ist, um den Ring (20) intern in die Funktions- oder Außerfunktionsposition wahlweise zu bringen ohne diesen axial zu verschieben.

10. Polyaxiale Pedikelschraube nach einem der Ansprüche 6 bis 9, **dadurch gekennzeichnet, dass** die Aufnahmehülse (8) und/oder der Stempel (24) zumindest im Bereich des Gegenkraftbauteils (20), vorzugsweise in Form des Spann- und/oder Klemmmittels und weiter vorzugsweise in Form des Spannrings mit federelastischen Eigenschaften ausgebildet ist, die zumindest in Radialrichtung wirken.

11. Polyaxiale Pedikelschraube nach Anspruch 10, **dadurch gekennzeichnet, dass** die Aufnahmehülse (8) und/oder der Stempel (24) mit Längsschlitzen (16) ausgeformt ist, wodurch elastisch radial nach außen aufbiegbare Laschen (18) entstehen, welche vom Gegenkraftbauteil (20) radial wahlweise zusammengehalten werden oder dass das Material der Aufnahmehülse (8) und/oder des Stempels (24) zumindest bereichsweise federelastisch radial aufweitbar ist.

12. Polyaxiale Pedikelschraube nach einem der Ansprüche 6 bis 11, **dadurch gekennzeichnet, dass** im Fall einer Axialverschiebbarkeit des Spannrings (20) zwei axial beabstandete Rastpositionen an der Aufnahmehülse (8) und/oder dem Stempel (24) ausgebildet sind, um die Funktions- oder Außerfunktionsposition zu markieren.

13. Polyaxiale Pedikelschraube nach einem der vorhergehenden Ansprüche, **dadurch gekennzeichnet, dass** das Gegenkraftbauteil (20) vorzugsweise manuell in eine erste, aktive Position in der das Verriegelungselement (32) seine Wirkung entfalten kann und in eine zweite, inaktive Position betätigbar ist, in der das Verriegelungselement (32) keine Wirkung entfalten kann.

14. Polyaxiale Pedikelschraube nach Anspruch 13, **dadurch gekennzeichnet, dass** das Gegenkraftbauteil in seiner ersten, aktiven Position Radialkräfte aufnimmt, um einer radialen Deformation der Aufnahmehülse (8) entgegen zu wirken und in seiner zweiten, inaktiven Position eine radiale Deformation der Aufnahmehülse (8) an einem bestimmten Axialabschnitt zulässt.

15. Wirbelkörper-Stabilisierungssystem bestehend aus zumindest einem Längsträger (30) zur longitudinalen Kopplung zweier Wirbelkörper und einer Anzahl von polyaxialen Pedikelschrauben (1) gemäß einem der Ansprüche 1 bis 14.

## Claims

1. A polyaxial pedicle screw (1) comprising a threaded shaft portion (2) for anchoring the pedicle screw (1) in a vertebra, said pedicle screw having one axial end provided with a shaft head (4) which is coupled to a mounting sleeve (8) for a longitudinal beam (30) in a rotatable and/or pivotable manner, comprising a fixing device (20, 32) for the selective positional fixation of the mounting sleeve (8) with respect to the shaft portion (2), **characterized in that** the fixing device (20, 32) comprises a locking element (32) as well as a separate counterforce part (20) counteracting the holding force of the locking element (32), preferably a grub screw or threaded nut, and being designed and/or retained in such a manner that the holding force of the locking element (32) can be selectively released by means of the counterforce part (20).

2. The polyaxial pedicle screw according to claim 1, **characterized in that** the locking element (32) is provided for a positional fixation of the adjusted polyaxial support if the counterforce part (20) is in a force-receiving state or in a functional position, whereas the counterforce part (20) is provided for neutralizing or reducing the function of the locking element (32) if the latter is already in a state of fixation.

3. The polyaxial pedicle screw according to claim 1 or 2, **characterized in that** the counterforce part (20) is designed as a manually destroyable disposable part and/or is provided with an opening mechanism by means of which the counterforce part (20) can be altered in terms of its dimensions and/or is movably supported such that it can be moved from a functional position to a non-functional position.

4. The polyaxial pedicle screw according to any of the claims 1 to 3, **characterized in that** the counterforce part (20) acts on the fixation mechanism of the fixing device for the positional fixation of the mounting sleeve (8) with respect to the shaft portion (2) and/or on the fixation mechanism of the fixing device for the positional fixation of the mounting sleeve (8) with respect to a longitudinal beam (30).

5. The polyaxial pedicle screw according to any of the claims 1 to 4, **characterized in that** the counterforce part (20) is designed as a tensioning or clamping device which is selectively able to maintain the shape and/or the function of the mounting sleeve (8) and/or of a punch (24) which is supported in the mounting sleeve (8) for transmitting a force from the locking element (32) to the shaft head (4).

6. The polyaxial pedicle screw according to any of the claims 1 to 5, **characterized in that** the counterforce part (20) is a tensioning ring which is preferably guided outside the mounting sleeve (8) and/or a punch (24) supported in the mounting sleeve so as to be axially movable therein and intended for transmitting a force between the locking element (32) and the shaft head (4) and is provided for applying a radially inward counterforce on the mounting sleeve (8) and/or the punch (24) depending on the adjusted shape and/or position, wherein the tensioning ring (20) is in particular made of a material which can be manually destroyed with a predetermined expenditure of force below the maximum load of the shaft portion (2) or is provided with a manually activatable predetermined breaking point.

7. The polyaxial pedicle screw according to any of the claims 1 to 5, **characterized in that** the counterforce part (20) is a threaded sleeve for receiving the locking element (32), which is selectively detachably latched in place in the mounting sleeve (8) preferably by a bayonet or clip lock, in order to transmit the preload force of the locking element (32) arranged in the threaded sleeve to the mounting sleeve (8) or to selectively release it.

8. The polyaxial pedicle screw according to claim 6, **characterized in that** the ring (20) is supported so as to be axially shiftable on the outer face of the mounting sleeve (8), so that it can be selectively moved to the functional or non-functional position.

9. The polyaxial pedicle screw according to claim 6, **characterized in that** the ring (20) is provided with an opening or width adjustment mechanism to selectively move the ring (20) internally to the functional or non-functional position without axially shifting it.

10. The polyaxial pedicle screw according to any of the claims 6 to 9, **characterized in that** the mounting sleeve (8) and/or the punch (24), at least in the area of the counterforce part (20), preferably in the form of the tensioning and/or clamping device and further preferably in the form of the tensioning ring, is/are provided with resilient features which act at least in the radial direction.

11. The polyaxial pedicle screw according to claim 10, **characterized in that** the mounting sleeve (8) and/or the punch (24) is/are realized with longitudinal slots (16), producing lugs (18) which can be elastically bent radially towards outside and are selectively kept together in the radial direction by the counterforce part (20), or **in that** the material of the mounting sleeve (8) and/or of the punch (24) is radially resiliently expandable at least in sections.

12. The polyaxial pedicle screw according to any of the claims 6 to 11, **characterized in that** two axially spaced latching positions are realized on the mounting sleeve (8) and/or on the punch (24) in order to mark the functional or non-functional position in case the tensioning ring (20) is axially shiftable.

13. The polyaxial pedicle screw according to any of the preceding claims, **characterized in that** the counterforce part (20) can be moved preferably manually to a first, active position in which the locking element (32) is able to develop its effect and to a second, inactive position, in which the locking element (32) is not able to develop any effect.

14. The polyaxial pedicle screw according to claim 13, **characterized in that** the counterforce part receives radial forces in its first, active position so as to counteract a radial deformation of the mounting sleeve (8), and in its second, inactive position it permits a radial deformation of the mounting sleeve (8) at a defined axial portion.

15. A vertebral body stabilizing system consisting of at least one longitudinal beam (30) for the longitudinal interconnection of two vertebral bodies, and a number of polyaxial pedicle screws (1) according to any of the claims 1 to 14.

## Revendications

1. Vis pédiculaire (1) polyaxiale avec une section de tige (2) vissée pour l'ancrage de la vis pédiculaire (1) dans une vertèbre, sur une extrémité axiale de laquelle une tête de tige (4) est réalisée, qui est couplée de manière rotative et/ou pivotante avec une douille de réception (8) pour un longeron (30), laquelle douille présente un moyen de fixation (20, 32) pour la fixation en position au choix de la douille de réception (8) par rapport à la section de tige (2), **caractérisée en ce que** le moyen de fixation (20, 32) a un élément de verrouillage (32) ainsi qu'un composant de force antagoniste (20) séparé qui agit contre la force de rappel de l'élément de verrouillage (32), de préférence une vis sans tête ou un écrou fileté et qui est conçu et/ou maintenu de sorte que la force de rappel de l'élément de verrouillage (32) soit libérable au choix au moyen du composant de force antagoniste (20).

2. Vis pédiculaire polyaxiale selon la revendication 1, **caractérisée en ce que** l'élément de verrouillage (32) est prévu pour une fixation en position de la polyaxialité réglée lorsque le composant de force antagoniste (20) se trouve dans une position fonctionnelle ou un état recevant la force, alors que le composant de force antagoniste (20) est prévu pour une suppression ou réduction de la fonction de l'élément de verrouillage (32) lorsque celui-ci se trouve déjà dans un état de fixation.

3. Vis pédiculaire polyaxiale selon la revendication 1 ou 2, **caractérisée en ce que** le composant de force antagoniste (20) est réalisé comme un composant à usage unique destructible manuellement et/ou est pourvu d'un mécanisme d'ouverture, par lequel le composant de force antagoniste (20) est modifiable par rapport à ses dimensions et/ou est logé de manière mobile de telle manière qu'il puisse être déplacé d'une position fonctionnelle à une position non fonctionnelle.

4. Vis pédiculaire polyaxiale selon l'une quelconque des revendications 1 à 3, **caractérisée en ce que** le composant de force antagoniste (20) agit sur le mécanisme de fixation du moyen de fixation pour la fixation en position de la douille de réception (8) par rapport à la section de tige (2) et/ou sur le mécanisme de fixation du moyen de fixation pour la fixation en position de la douille de réception (8) par rapport à un longeron (30).

5. Vis pédiculaire polyaxiale selon l'une quelconque des revendications 1 à 4, **caractérisée en ce que** le composant de force antagoniste (20) est réalisé comme un moyen de tension ou de serrage qui est en mesure au choix de conserver la forme et/ou la fonction de la douille de réception (8) et/ou d'un poinçon (24) qui est logé pour la transmission de force de l'élément de verrouillage (32) sur la tête de tige (4) dans la douille de réception (8).

6. Vis pédiculaire polyaxiale selon l'une quelconque des revendications 1 à 5, **caractérisée en ce que** le composant de force antagoniste (20) est un anneau de serrage qui est guidé de préférence côté extérieur autour de la douille de réception (8) et/ou d'un poinçon (24) logé de manière mobile axialement dans la douille de réception pour la transmission de force entre l'élément de verrouillage (32) et la tête de tige (4) et est prévu afin d'appliquer selon la forme et/ou la position réglée une force antagoniste dirigée radialement vers l'intérieur sur la douille de réception (8) et/ou le poinçon (24), dans laquelle l'anneau de serrage (20) est fabriqué en particulier en un matériau destructible manuellement avec une dépense de force prédéterminée sous la limite de sollicitation de la section de tige (2) ou est réalisé avec un point destiné à la rupture actionnable manuellement.

7. Vis pédiculaire polyaxiale selon l'une quelconque des revendications 1 à 5, **caractérisée en ce que** le composant de force antagoniste (20) est une douille filetée pour la réception de l'élément de verrouillage (32) qui est encliquetée dans la douille de réception (8) au choix de manière détachable de préférence par une fermeture à baïonnette ou clip afin de transmettre ou libérer au choix la force de précontrainte de l'élément de verrouillage (32) agencé dans la douille filetée sur la douille de réception (8).

8. Vis pédiculaire polyaxiale selon la revendication 6, **caractérisée en ce que** l'anneau (20) est logé de manière axialement mobile sur le côté extérieur de la douille de réception (8) afin de pouvoir être amené au choix dans la position fonctionnelle ou non fonctionnelle.

9. Vis pédiculaire polyaxiale selon la revendication 6, **caractérisée en ce que** l'anneau (20) est pourvu d'un mécanisme de réglage d'ouverture ou de largeur afin d'amener au choix l'anneau (20) en interne dans la position fonctionnelle ou non fonctionnelle à se déplacer axialement.

10. Vis pédiculaire polyaxiale selon l'une quelconque des revendications 6 à 9, **caractérisée en ce que** la douille de réception (8) et/ou le poinçon (24) est réalisé au moins dans la zone du composant de force antagoniste (20), de préférence sous la forme du moyen de tension et/ou de serrage et encore de préférence sous la forme de l'anneau de serrage avec des propriétés élastiques qui agissent au moins dans le sens radial.

11. Vis pédiculaire polyaxiale selon la revendication 10, **caractérisée en ce que** la douille de réception (8) et/ou le poinçon (24) est formé avec des fentes longitudinales (16), moyennant quoi des languettes (18) pliables élastiquement radialement vers l'extérieur apparaissent, lesquelles sont maintenues radialement au choix par le composant de force antagoniste (20) ou **en ce que** le matériau de la douille de réception (8) et/ou du poinçon (24) peut être élargi radialement élastiquement au moins par endroits.

12. Vis pédiculaire polyaxiale selon l'une quelconque des revendications 6 à 11, **caractérisée en ce que** dans le cas d'une mobilité axiale de l'anneau de serrage (20), deux positions d'encliquetage espacées axialement sont réalisées sur la douille de réception (8) et/ou le poinçon (24) afin de marquer la position fonctionnelle ou non fonctionnelle.

13. Vis pédiculaire polyaxiale selon l'une quelconque des revendications précédentes, **caractérisée en ce que** le composant de force antagoniste (20) peut être actionné de préférence manuellement dans une première position active, dans laquelle l'élément de verrouillage (32) peut déployer son action et dans une seconde position inactive, dans laquelle l'élément de verrouillage (32) ne peut déployer aucune action.

14. Vis pédiculaire polyaxiale selon la revendication 13, **caractérisée en ce que** le composant de force antagoniste reçoit dans sa première position active des forces radiales afin d'agir contre une déformation radiale de la douille de réception (8) et autorise dans sa seconde position inactive une déformation radiale de la douille de réception (8) sur une section axiale déterminée.

15. Système de stabilisation de corps de vertèbre constitué d'au moins un longeron (30) pour le couplage longitudinal de deux corps de vertèbre et d'un nombre de vis pédiculaires polyaxiales (1) selon l'une quelconque des revendications 1 à 14.
